# FASCICULE DE BREVET EUROPEEN

(11) **EP 1 268 398 B1**
(45) Date de publication et mention de la délivrance du brevet: **08.06.2005**
(21) Numéro de dépôt: 01921440.2
(22) Date de dépôt: 30.03.2001
(51) Int. Cl.: C07C 227/32, C07D 209/34, C07C 229/16

(54) **NOUVEAU PROCEDE DE SYNTHESE DES ESTERS DE LA N- (S)-1-CARBOXYBUTYL]-(S)-ALANINE ET APPLICATION A LA SYNTHESE DU PERINDOPRIL**
VERFAHREN ZUR HERSTELLUNG VON N-[(S)-1-CARBOXYBUTYL]-(S) ALANINE ESTERN UND VERWENDUNG ZUR SYNTHESE VON PERINDOPRIL
NOVEL METHOD FOR SYNTHESIS OF N- (S)-1-CARBOXYBUTYL]-(S)-ALANINE ESTERS AND USE IN SYNTHESIS OF PERINDOPRIL

(30) Priorité: 31.03.2000 FR 0004112
(43) Date de publication de la demande: 02.01.2003
(73) Titulaire: Laboratoires Servier, 92415 Courbevoie Cedex (FR)
(72) Inventeur: SOUVIE, Jean-Claude, F-76600 Le Havre (FR)
(86) Numéro de dépôt international: PCT/FR2001/000959
(87) Numéro de publication internationale: WO 2001/056353

(56) Documents cités:
- EP-A- 0 308 340

## Description

La présente invention concerne un procédé de synthèse industrielle des esters de la N-[(S)-1-carboxybutyl]-(S)-alanine, et leur application à la synthèse industrielle du perindopril et de ses sels pharmaceutiquement acceptables.

Plus spécifiquement, la présente invention concerne un nouveau procédé de synthèse industrielle des dérivés de formule (I) : 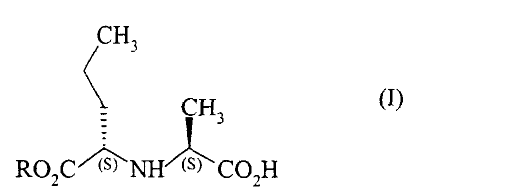 dans laquelle R représente un groupement alkyle (C₁-C₆) linéaire ou ramifié,
ainsi que leurs sels d'addition à un acide ou à une base minéral(e) ou organique.

Les composés de formule (I) obtenus selon le procédé de l'invention sont utiles dans la synthèse du perindopril de formule (II) : 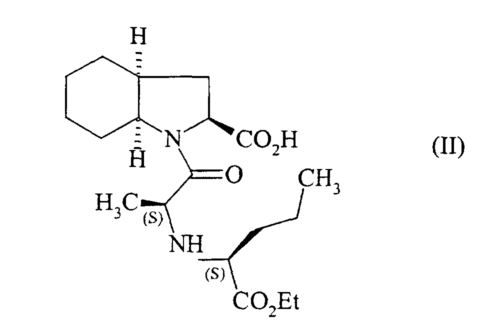 ainsi que dans celle de ses sels pharmaceutiquement acceptables.

Le perindopril, ainsi que ses sels, possèdent des propriétés pharmacologiques intéressantes. Leur principale propriété est d'inhiber l'enzyme de conversion de l'angiotensine I (ou kininase 11), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse industrielle performant, permettant notamment l'obtention sélective du diastéréoisomère (S,S) avec un bon rendement et une excellente pureté, mais également facilement transposable à l'échelle industrielle.

Quelques méthodes de préparation des composés de formule (I) sont déjà connues. Toutefois, ces procédés présentent des inconvénients importants à l'échelle industrielle :
- Le journal Tet. Lett. 1982, 23 (16), 1677-80 décrit l'obtention d'un dérivé de formule (I) (R = éthyle) par réaction dans l'éthanol du 2-oxovalérate d'éthyle avec l'ester tert-buylique de l'alanine en présence de cyanoborohydrure de sodium. Mais cet agent réducteur est particulièrement toxique, très hygroscopique et difficilement manipulable à l'échelle industrielle.
- Le brevet EP 0 309 324 décrit l'accès à un composé de formule (I) (R = éthyle) par réaction dans le diméthylformamide de l'ester benzylique de l'alanine avec l'α-bromovalérate d'éthyle en présence de triéthylamine. Les inconvénients majeurs de ce procédé sont le nombre important d'étapes et le faible rendement en isomère (S,S). En effet, la réaction n'étant pas diastéréosélective, on est obligé, pour accéder à l'isomère (S,S) pur, de rajouter une étape de purification par cristallisation fractionnée en présence d'acide maléique.
- Les brevets EP 0 308 340 et EP 0 308 341 décrivent l'accès à un composé de formule (I) (R = éthyle) par réaction dans l'eau du chlorhydrate de norvalinate d'éthyle avec l'acide pyruvique en présence d'hydrogène, de charbon palladié et de soude.
   L'isolement du produit brut se fait ensuite par évaporation de l'eau, puis de l'éthanol est ajouté pour précipiter le chlorure de sodium formé pendant la réaction. Après filtration, la solution éthanolique obtenue est évaporée et le résidu est recristallisé dans l'acétonitrile.
   Ce procédé présente l'avantage de conduire au composé de formule (I) avec une excellente pureté optique : seul le diastéréoisomère (S, S) cristallise dans ces conditions. De plus, l'utilisation comme réactif de l'acide pyruvique, produit naturel, peu coûteux et industriellement disponible, et celle de l'eau comme solvant de réaction, est particulièrement avantageuse.
   Par contre, ce procédé présente aussi un inconvénient qui rend sa mise en oeuvre particulièrement laborieuse à l'échelle industrielle : l'isolement du produit de réaction passe en effet par l'évaporation d'une grande quantité d'eau, puis nécessite une série d'opérations (ajout d'un premier solvant organique, filtration, évaporation puis recristallisation dans un deuxième solvant organique) pour arriver au produit chimiquement et optiquement pur.

La demanderesse a présentement mis au point un nouveau procédé de synthèse industrielle des dérivés de formule (I), qui combine les avantages de la réaction d'hydrogénation en milieu aqueux avec un isolement particulièrement rapide et simple à mettre en oeuvre à l'échelle industrielle.

Plus spécifiquement, la présente invention concerne un procédé de synthèse industrielle des composés de formule (I) caractérisé en ce que l'on condense le pyruvate de sodium de formule (III) : 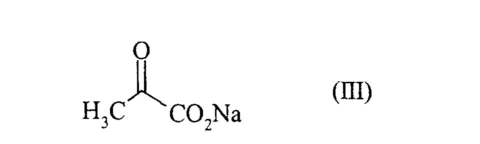 avec un composé de formule (IV) : 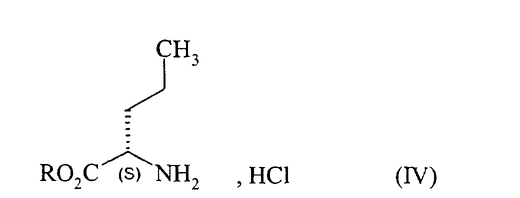 dans laquelle R a la même signification que dans la formule (I),
sous hydrogénation catalysée par le charbon palladié à 5 %,
dans l'eau,
sous une pression comprise entre 1 et 20 bars, préférentiellement entre 1 et 5 bars,
à une température comprise entre 10 et 60°C, préférentiellement entre 10 et 40°C,
en présence d'une quantité de soude comprise entre 0,1 et 0,2 mole par mole de dérivé de formule (IV) utilisé, pour conduire directement, après acidification du milieu réactionnel à pH compris entre 2,8 et 4,5, préférentiellement entre 3 et 3,5, suivie d'une filtration, au dérivé de formule (I) optiquement pur.
- Une pression faible en hydrogène permet de façon inattendue d'obtenir un rendement et une pureté chimique et énantiomérique aussi bons qu'en effectuant la réaction à pression élevée.
- De manière surprenante, la simple précipitation après acidification du milieu réactionnel aqueux conduit au seul isomère (S, S) avec une bonne pureté chimique et une excellente pureté énantiomérique.
   L'étape de recristallisation peut donc être supprimée, ce qui rend l'isolement particulièrement rapide et simple à mettre en oeuvre à l'échelle industrielle.

L'exemple ci-dessous illustre l'invention, mais ne la limite en aucune façon.

### Exemple : N-[(S)-Carbéthoxy-1-butyl]-(S)-alanine

Dans une cuve équipée d'un agitateur, placer 3 kg de chlorhydrate de S-norvalinate d'éthyle en solution dans l'eau, 0.6 1 d'une solution aqueuse d'hydroxyde de sodium 4N et 2 kg de pyruvate de sodium. Dans un appareil à hydrogéner, placer du charbon palladié à 5 % en suspension dans l'eau, puis la solution précédemment obtenue. Hydrogéner sous pression de 1,2 bar à 35°C jusqu'à absorption de la quantité théorique d'hydrogène. Eliminer le catalyseur par filtration, puis ajouter au filtrat de l'acide chlorhydrique concentré jusqu'à pH = 3,1. Refroidir entre 0 et 5°C, puis récolter le solide obtenu par filtration. Laver le gâteau par l'acétonitrile glacé et sécher en étuve ventilée à 40°C jusqu'à poids constant.
La N-[(S)-Carbéthoxy-1-butyl]-(S)-alanine est ainsi obtenue avec un rendement de 62%, une pureté chimique de 95% et une pureté énantiométrique supérieure à 99%.

## Revendications

1. Procédé de synthèse industrielle des composés de formule (I) 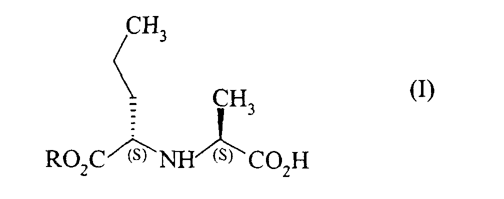 dans laquelle R représente un groupement alkyle (C₁-C₆) linéaire ou ramifié, **caractérisé en ce que** l'on condense le pyruvate de sodium de formule (III) : 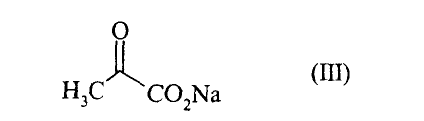 avec un composé de formule (IV) : 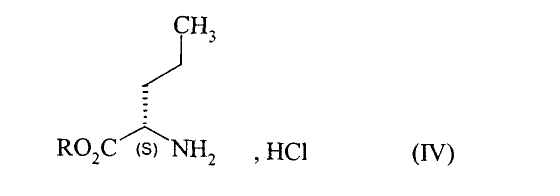 dans laquelle R a la même signification que dans la formule (I),
sous hydrogénation catalysée par le charbon palladié à 5 %,
dans l'eau,
sous une pression comprise entre 1 et 20 bars,
à une température comprise entre 10 et 60°C, en présence d'une quantité de soude comprise entre 0,1 et 0,2 mole par mole de dérivé de formule (IV) utilisé, pour conduire directement, après acidification du milieu réactionnel à pH compris entre 2,8 et 4,5, suivie d'une filtration, au dérivé de formule (I) optiquement pur.

2. Procédé de synthèse selon la revendication 1, permettant l'obtention du dérivé de formule (I) dans laquelle R représente le groupement éthyle.

3. Procédé de synthèse selon l'une quelconque des revendications 1 ou 2, **caractérisé en ce que** la pression d'hydrogénation est comprise entre 1 et 5 bars.

4. Procédé de synthèse selon l'une quelconque des revendications 1 à 3, **caractérisé en ce que** la température d'hydrogénation est comprise entre 10 et 40°C.

5. Procédé de synthèse selon l'une quelconque des revendications 1 à 4, **caractérisé en ce que** le pH après acidification est compris entre 3 et 3,5.

6. Procédé de synthèse du perindopril ou de ses sels pharmaceutiquement acceptables **caractérisé en ce que** l'on prépare un dérivé de formule (I): 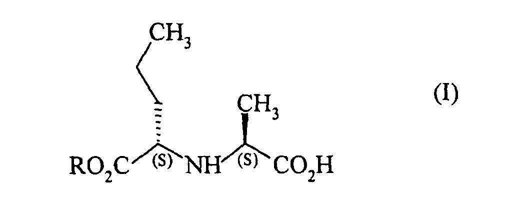 dans laquelle R représente un groupement alkyle (C₁-C₆) linéaire ou ramifié, selon le procédé de l'une quelconque des revendications 1 à 5.

## Claims

1. Process for the industrial synthesis of the compounds of formula (I) 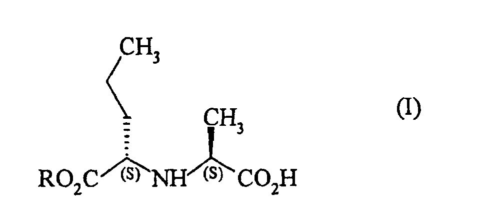 wherein R represents a linear or branched (C₁-C₆)alkyl group, **characterised in that** sodium pyruvate of formula (III) : 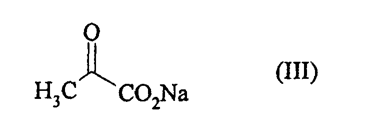 is condensed with a compound of formula (IV) : 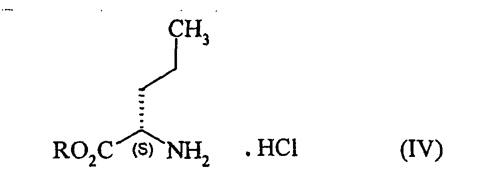 wherein R is as defined for formula (I),
with hydrogenation catalysed by 5 % palladium-on-carbon,
in water,
at a pressure of from 1 to 20 bar,
at a temperature of from 10 to 60°C, in the presence of sodium hydroxide in an amount of from 0.1 to 0.2 mol per mol of compound of formula (IV) used, to yield the compound of formula (I) directly, in optically pure form, following acidification of the reaction mixture to a pH of from 2.8 to 4.5 and then filtration.

2. Synthesising process according to claim 1 that allows the compound of formula (I) wherein R represents an ethyl group to be obtained.

3. Synthesising process according to either claim 1 or claim 2, **characterised in that** the hydrogenation pressure is from 1 to 5 bar.

4. Synthesising process according to any one of claims 1 to 3, **characterised in that** the hydrogenation temperature is from 10 to 40°C.

5. Synthesising process according to any one of claims 1 to 4, **characterised in that** the pH after acidification is from 3 to 3.5.

6. Process for the synthesis of perindopril or its pharmaceutically acceptable salts, **characterised in that** a compound of formula (I) 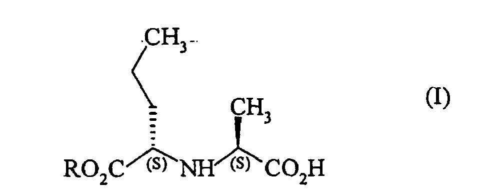 wherein R represents a linear or branched (C₁-C₆)alkyl group is prepared in accordance with the process of any one of claims 1 to 5.

## Patentansprüche

1. Verfahren zur technischen Synthese der Verbindungen der Formel (I): 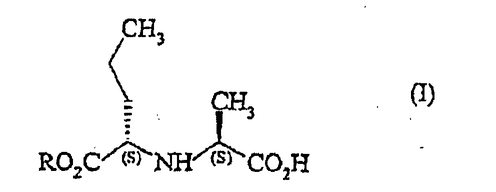 in der R eine geradkettige oder verzweigte (C₁-C₆)-Alkylgruppe bedeutet, **dadurch gekennzeichnet, daß** man Natriumpyruvat der Formel (III): 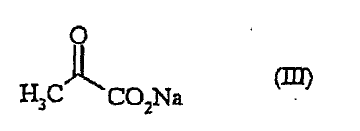 mit einer Verbindung der Formel (IV): 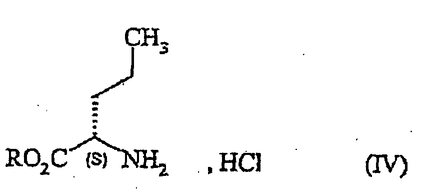 in der R die bezüglich der Formel (I) angegebenen Bedeutungen besitzt, unter Hydrierung durch Katalyse durch 5% Palladium-auf-Kohlenstoff, in Wasser,
bei einem Druck zwischen 1 und 20 bar,
bei einer Temperatur zwischen 10 und 60°C
in Gegenwart einer Natriumhydroxidmenge zwischen 0,1 und 0,2 Mol pro Mol des verwendeten Derivats der Formel (IV) kondensiert, so daß man direkt nach dem Ansäuern des Reaktionsmediums auf einen pH-Wert zwischen 2,8 und 4,5, gefolgt von einer Filtration, das optisch reine Derivat der Formel (I) erhält.

2. Syntheseverfahren nach Anspruch 1 für die Herstellung des Derivats der Formel (I), in der R die Ethylgruppe bedeutet.

3. Syntheseverfahren nach einem der Ansprüche 1 oder 2, **dadurch gekennzeichnet, daß** der Wasserstoffdruck zwischen 1 und 5 bar beträgt.

4. Syntheseverfahren nach einem der Ansprüche 1 bis 3, **dadurch gekennzeichnet, daß** die Temperatur der Hydrierung zwischen 10 und 40°C beträgt.

5. Syntheseverfahren nach einem der Ansprüche 1 bis 4, **dadurch gekennzeichnet, daß** der pH-Wert nach dem Ansäuern zwischen 3 und 3,5 beträgt.

6. Verfahren zur Synthese von Perindopril oder von seinen pharmazeutisch annehmbaren Salzen, **dadurch gekennzeichnet, daß** man ein Derivat der Formel (I): 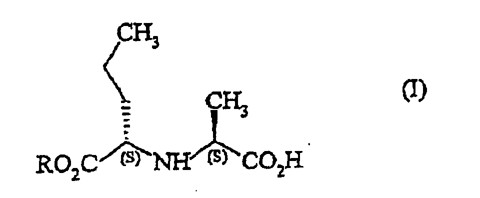 in der R eine geradkettige oder verzweigte (C₁-C₆)-Alkylgruppe bedeutet, nach dem Verfahren nach einem der Ansprüche 1 bis 5 herstellt.
